# EUROPÄISCHE PATENTANMELDUNG

(11) **EP 2 208 508 A1**
(43) Veröffentlichungstag der Anmeldung: **21.07.2010**
(21) Anmeldenummer: 09172779.2
(22) Anmeldetag: 12.10.2009
(51) Int. Cl.: A61N 5/10

(54) **Bewegungsdetektion mittels Radarsystem während einer Bestrahlung**

(30) Priorität: 19.01.2009 DE 102009005110
(71) Anmelder: Siemens Aktiengesellschaft, 80333 München (DE)
(72) Erfinder: Saar, Matthias, 96146, Altendorf (DE)

(57) **Zusammenfassung**

Die Erfindung betrifft eine Vorrichtung zur Bestrahlung eines Patienten (P) mit einer auf den Patienten (P) ausgerichteten Bestrahlungsquelle, sowie mit einem Radarsystem (S1, E1, S2, E2) zur Erfassung der Position des Patienten (P) oder eines Teils (T) des Patienten (P) während der Bestrahlung.

## Beschreibung

Die Erfindung betrifft eine Vorrichtung zur Bestrahlung eines Patienten mit einer auf den Patienten ausgerichteten Bestrahlungsquelle.

In der Partikel-Therapie, einer Therapieform bei Krebserkrankungen, wird ein Tumor hochpräzise mit einem Protonen- oder Ionenstrahl bestrahlt. Im Gegensatz zu einer Bestrahlung mit elektromagnetischen Strahlen wird bei einer Ionenbestrahlung der Großteil der Strahlenergie direkt im Tumor entladen. Das darüberliegende Gewebe wird sehr viel weniger geschädigt. Die Heilungschancen sind nicht schlechter als bei einer Therapie mit anderen Strahlen, aber es entstehen weitaus weniger Nebenwirkungen. Vielfach werden Kohlenstoff- und Neonionen verwendet.

Um möglichst wenig gesundes Gewebe zu schädigen, ist es wichtig, den Patienten so zu lagern, dass die Partikel mit der gewünschten Körperregion, d.h. mit dem Tumorgewebe, wechselwirken, und möglichst wenig mit dem gesunden Gewebe. Eine Positionsänderung des Patienten führt i.d.R. dazu, dass diese Bedingung nicht mehr eingehalten ist. Selbiges gilt auch für die Atem- und Herztätigkeit des Patienten, falls die zu bestrahlende Region im Brust- oder Bauchraum liegt. Dies führt zu einer unerwünschten Bestrahlung gesunden Gewebes.

Der Erfindung liegt die Aufgabe zugrunde, eine Vorrichtung zur Bestrahlung eines Patienten aufzuzeigen, welche zur Vermeidung des geschilderten negativen Effektes der Bestrahlung gesunden Gewebes beiträgt. Ferner sollen ein entsprechendes Computerprogramm und ein Verfahren vorgestellt werden.

Diese Aufgabe wird durch eine Vorrichtung mit den Merkmalen des Anspruchs 1, sowie durch ein Verfahren und ein Computerprogramm mit Merkmalen von nebengeordneten Ansprüchen gelöst. Vorteilhafte Ausgestaltungen und Weiterbildungen sind Gegenstand von Unteransprüchen.

Die erfindungsgemäße Vorrichtung zur Bestrahlung eines Patienten umfasst eine auf den Patienten ausgerichtete Bestrahlungsquelle und eines oder mehrere Radarsysteme zur Erfassung der Position des Patienten oder eines Teils des Patienten während der Bestrahlung.

Von einem Radarsystem oder -gerät werden elektromagnetische Wellen als so genanntes Primärsignal ausgesendet und die von Objekten reflektierten "Echos" empfangen. Durch eine Auswertung der empfangenen Echos können Informationen über das reflektierende Objekt gewonnen werden.

Der Einsatz des Radarsystems bzw. der Radarsysteme dient der Erfassung einer Patientenposition. Hierbei kann es sich um eine absolute oder eine relative Position handeln, z.B. relativ zur Bestrahlungsquelle oder dem zumindest einen Radarsystem. Es kann die Position oder Lage des gesamten Patienten von Interesse sein oder auch nur eines Teils des Patienten, wie z.B. eines Organs oder Tumors. Die Positionserfassung ist insbesondere im Zusammenspiel mit der Bestrahlung von Vorteil, denn bei einer Bestrahlung ist es nötig zu wissen, welchen Körperteil des Patienten der Strahl trifft.

Die Positionserfassung erfolgt während der Bestrahlung des Patienten. Dies impliziert, dass das oder die Radarsysteme derart ausgestaltet und/oder orientiert sind, dass sie durch die Bestrahlung keine Beschädigung erfahren. Die Erfassung während der Bestrahlung bedeutet insbesondere, dass die Bestrahlung nicht unterbrochen werden muss, um die Positionserfassung mittels des Radarsystems bzw. der Radarsysteme durchzuführen. Von Vorteil hierbei ist die Möglichkeit der unterbrechungsfreien Anpassung der Bestrahlung an eine geänderte Position des Patienten oder der bestrahlten Körperregion des Patienten.

Neben den beschriebenen Bestandteilen kann die erfindungsgemäße Vorrichtung weitere Bestandteile aufweisen, von welchen manche bei den folgenden Ausgestaltungen und Weiterbildungen näher erläutert werden.

Bei dem mindestens einen Radarsystem handelt es sich vorzugsweise um ein UWB-Radarsystem. Diese zeichnen sich durch eine gute örtliche Auflösung aus. Außerdem sind die von einem solchen Radargerät ausgestrahlten Frequenzen für den menschlichen Körper ungefährlich.

In Weiterbildung der Erfindung ist eine Auswerteeinheit vorhanden zur Ermittlung der Position des Patienten oder des Teils des Patienten aus von dem mindestens einen Radarsystem erfassten Daten. Die Auswerteeinheit kann geeignete Algorithmen anwenden, um aus den von dem mindestens einen Radarsystem gelieferten Informationen die gewünschte Position zu berechnen. Insbesondere kann die Auswerteeinheit dazu genutzt werden, Änderungen dieser Position festzustellen.

Einer Ausgestaltung der Erfindung gemäß ist die Bestrahlungsquelle eine Partikel emittierende Quelle. Bei den Partikeln handelt es sich vorzugsweise um Ionen wie beispielsweise Kohlenstoffionen, oder um Protonen.

Vorteilhaft ist es, wenn nicht nur eines, sondern zwei Radarsysteme eingesetzt werden. Diese können ihre Primärstrahlung in unterschiedlichen Winkeln, d.h. nicht parallel zueinander, auf den Patienten versenden. Hierdurch wird die örtliche Auflösung erhöht.

In Weiterbildung der Erfindung ist eine Steuereinrichtung vorhanden zum Nachführen und/oder Unterbrechen des von der Bestrahlungsquelle emittierten Strahls aufgrund der erfassten Position des Patienten oder des Teils des Patienten. Vorzugsweise ist die Steuereinrichtung an die Auswerteeinheit angeschlossen und steuert den Strahl in Abhängigkeit von Ergebnissen der Auswerteeinheit. Das Nachführen des Strahls entspricht einer Richtungsänderung, während das Unterbrechen ein zumindest zeitweiliges Abschalten des Strahls darstellt. Derartige Maßnahmen sind insbesondere bei festgestellten Positionsänderungen des Patienten oder von Organen des Patienten von Vorteil. Hierdurch kann vermieden werden, dass der Patient in unerwünschten Regionen bestrahlt wird. Durch ein geeignetes Nachfahren des Strahls ist es möglich, trotz Positionsänderungen des Patienten oder des Teils des Patienten konstant den gleichen Körperausschnitt zu bestrahlen.

Zusätzlich oder alternativ zur oben erwähnten Auswerteeinheit zur Ermittlung der Position des Patienten oder des Teils des Patienten kann eine Auswerteeinheit vorhanden sein zur Ermittlung der Position einer anderen Person als des Patienten und/oder eines Gerätes aus von dem mindestens einen Radarsystem erfassten Daten. Als andere Person kommt hierbei medizinisches Personal in Betracht, welche die Bestrahlung bzw. den Patienten während der Bestrahlung überwachen. Es kann somit überprüft werden, ob sich Personen oder Geräte an gefährlichen oder unerwünschten Orten innerhalb des Bestrahlungsraumes befinden oder sich auf solche Orte hinbewegen. Insbesondere kann hierdurch geprüft bzw. festgestellt werden, ob sich andere Personen innerhalb des Bestrahlungsraums befinden. Denn während der Bestrahlung sollte sich außer dem Patienten niemand im Behandlungsraum aufhalten.

Vorteilhaft ist eine Steuereinrichtung zum Nachführen und/oder Unterbrechen des von der Bestrahlungsquelle emittierten Strahls aufgrund der erfassten Position der Person und/oder des Gerätes. Eine Unterbrechung ist z.B. dann sinnvoll, wenn sich die Person oder das Gerät nahe dem Strahl oder der Strahlaustrittsöffnung befindet. Hier können sich empfindliche elektronische Messeinrichtungen befinden, die nicht berührt werden dürfen. Zusätzlich oder alternativ zur Beeinflussung des Strahls durch Unterbrechen und/oder Nachführen ist es möglich, dass aufgrund der erfassten Position der Person und/oder des Gerätes die Bewegung des Gerätes geändert oder gestoppt wird, oder eine Abschaltung von Elektronikeinheiten des Gerätes erfolgt.

Zusätzlich oder alternativ zu den beiden oben erwähnten Auswerteeinheiten kann eine Auswerteeinheit vorhanden sein zur Ermittlung von einer Atem- und/oder Herztätigkeit des Patienten aus von dem mindestens einen Radarsystem erfassten Daten. Dies ermöglicht es, auf andere Geräte zur Beobachtung von Atmung und Puls des Patienten während der Bestrahlung ganz oder teilweise zu verzichten.

Vorteilhaft ist eine Steuereinrichtung zum Nachführen und/oder Unterbrechen des von der Bestrahlungsquelle emittierten Strahls aufgrund der ermittelten Atem- und/oder Herztätigkeit. Eine Unterbrechung ist z.B. dann sinnvoll, wenn sich anhand der hierfür charakteristischen Atem- und/oder Herztätigkeit ein Panik- oder Unruhezustand des Patienten erkennen lässt.

Die mehreren beschriebenen Auswerteeinheiten können unabhängig voneinander realisiert werden. Es ist jedoch auch möglich, dass eine einzige Auswerteeinheit vorhanden ist, welche eine, mehrere oder alle der erläuterten Ermittlungsfunktionen wahrnimmt.

Das erfindungsgemäße Computerprogramm verfügt über die Funktionalität eines Eingangs für Daten von zumindest einem Radarsystem zur Erfassung der Position eines Patienten oder eines Teils des Patienten während einer Bestrahlung, sowie über die Funktionalität eines Auswertestandteils zur Ermittlung der Position des Patienten oder des Teils des Patienten aus den Daten.

Vorteilhaft ist ferner die Funktionalität eines Steuerbestandteils zum Steuern des Nachführens und/oder Unterbrechens des von der Bestrahlungsquelle emittierten Strahls aufgrund der ermittelten Position des Patienten oder des Teils des Patienten.

Ferner ist die Funktionalität eines Auswertebestandteils möglich zur Ermittlung der Position einer Person und/oder eines Gerätes und/oder zur Ermittlung von einer Atem- und/oder Herztätigkeit des Patienten aus den Daten.

Bei dem erfindungsgemäßen Positionserfassungsverfahren wird zumindest ein Radarsystem zur Erfassung der Position eines Patienten oder eines Teils des Patienten während einer Bestrahlung eingesetzt.

Die Ausführungen betreffend die Vorteile, Ausgestaltungen und Weiterbildungen der erfindungsgemäßen Vorrichtung gelten entsprechend auf für das erfindungsgemäße Computerprogramm und das erfindungsgemäße Verfahren. Hierzu können sie weitere geeignete Funktionalitäten bzw. Schritte umfassen.

Im folgenden wird die Erfindung anhand eines Ausführungsbeispiels näher erläutert. Dabei zeigt:

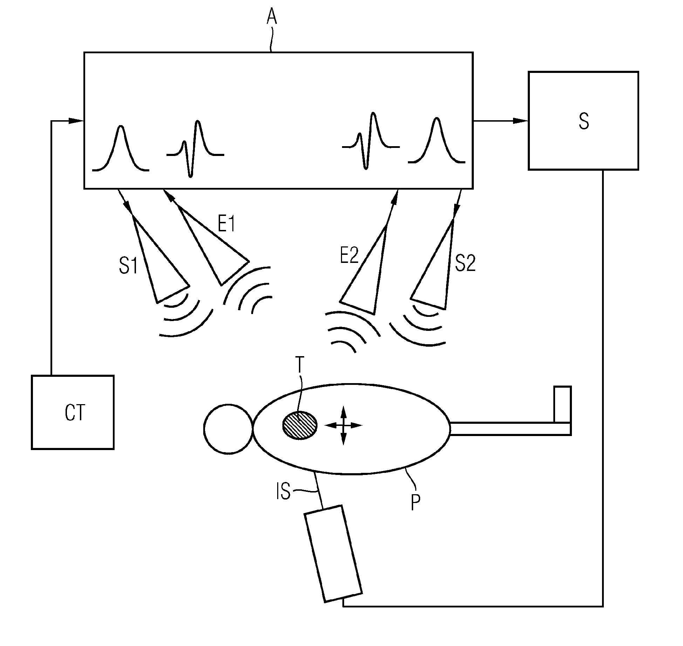

- Figur 1:: ein Überwachungssystem.

In Figur 1 ist ein Patient P zu sehen, welcher im Rahmen einer Partikel-Therapie mittels eines Ionenstrahls IS bestrahlt wird. Hierbei kann es sich z.B. um einen Strahl aus Kohlenstoff-Ionen handeln. Auch ein Protonenstrahl kann zum Einsatz kommen. Ziel der Bestrahlung ist es, dass der Ionenstrahl IS den Tumor T des Patienten erreicht und diesen zerstört. Die Steuereinrichtung S dient der Steuerung der Ablenkung des Ionenstrahls IS in verschiedene Richtungen; unter Einsatz von - nicht dargestellten - Magneten kann durch die Steuereinrichtung S die Richtung des Ionenstrahls IS geändert werden, so dass er genau auf den Tumor gerichtet werden kann. Diese Ablenkbarkeit des Ionenstrahls IS ist durch die beiden Doppelpfeile neben dem Tumor T veranschaulicht.

Bei der Bestrahlung ist es wichtig, dass der Ionenstrahl IS exakt auf den Tumor T ausgerichtet ist und das dem Tumor T entsprechende Zielvolumen trifft. Eine Verschiebung des Ionenstrahls IS um nur wenige Millimeter würde Teile des gesunden Gewebes zerstören und Teile des Tumors T überleben lassen, wodurch ein Wiederaufleben des Tumors T gefördert würde. Es ist daher bei der Bestrahlung erforderlich, die Position des Tumors T möglichst genau zu kennen.

Hierzu wird zunächst der Patient P in einem Immobilisierungsraum auf einer Tischplatte fixiert und dann mittels eines Shuttle-Systems in den Behandlungsraum transportiert. Eine roboterbasierte Patientenlagerungsvorrichtung übernimmt die Tischplatte mit dem Patienten P und führt diese präzise in die gewünschte Lage. Da zuvor mittels eines bildgebenden Verfahrens wie z.B. der Computertomographie die Lage des Tumors T innerhalb des Patienten P genau ermittelt wurde, ist hiermit die Position des Tumors T bekannt.

Ferner ist im Behandlungsraum, z.B. an der Decke, eine Röntgenanlage CT zur Verifizierung der Position des Tumors T vorhanden. Stimmt die mit der Röntgenanlage CT erfasste Position nicht mit der Zielposition überein, regelt der Tischroboter nach, bis der Tumor T genau im Isozentrum liegt.

Diese Röntgenanlage CT wird jedoch während der Bestrahlung mit dem Ionenstrahl IS nicht betrieben. Vielmehr wird sie vor der Bestrahlung hoch an die Decke geschwenkt, um möglichst weit weg von der Primärstrahlung zu sein. Dies bedeutet, dass während der Bestrahlung nicht die Möglichkeit besteht, mittels Computertomographie die Position des Tumors T zu überprüfen.

Erfolgt die Bestrahlung anders als in Figur 1 dargestellt am Kopf des Patienten P, kann eine Patientenmaske zum Einsatz kommen, welche den Kopf des Patienten fixiert. Eine Kopfbewegung des Patienten P mit dieser Maske ist kaum möglich, so dass eine Vorbeitreffen des Ionenstrahls IS am Tumor T fast ausgeschlossen ist.

Wenn sich hingegen der Tumor T wie in Figur 1 dargestellt im Bauch- oder Brustraum des Patienten P befindet, so verschiebt er sich trotz äußerer Fixierung des Patienten P durch dessen Atem- und Herzbewegungen. Diese Bewegungen des Tumors T führen, sofern der Ionenstrahl IS ihnen nicht angepasst wird, zu einer Fehlbestrahlung des Patienten P.

Man kann versuchen, die Atem- und Herzbewegungen des Patienten P zu erfassen und hieraus Rückschlüsse auf die Bewegung des Tumors T zu ziehen. Möglichkeiten hierfür bieten z.B. Lasersysteme, die auf der Haut des Patienten angebrachte Lasermarken vermessen, oder Brustgurte. Nachteilig ist, dass sich hieraus allenfalls indirekte Informationen über die Bewegungen des Tumors T ergeben. Denn die Bewegung der Oberfläche des Patienten P stimmt i.d.R. nicht mit derjenigen des Tumors T überein.

Um die Position des Tumors T, welche sowohl von der - eventuell veränderlichen - Lage des Patienten P, als auch von seiner Atem- und Herztätigkeit abhängt, während der Bestrahlung zu überprüfen, werden zwei Ultra-Wideband (UWB) Radarsysteme eingesetzt. Das erste Radarsystem umfasst den Sender S1 und den Empfänger E1, das zweite den Sender S2 und den Empfänger E2. Diese sind an die Auswerteeinheit A angeschlossen. Der Empfänger E1 dient der Erfassung der von dem Sender S1 ausgestrahlten und von dem Patienten P bzw. seiner Umgebung reflektierten Strahlung; entsprechendes gilt für den Empfänger E2 in Bezug auf die Strahlung des Senders S2.

UWB-Systeme nutzen die Eigenschaften elektromagnetischer Felder mit extrem großer Bandbreite; diese beträgt ca. 1-10 GHz im Frequenzbereich von 3 - 11 GHz. Die Sendeleistung ist niedrig, d.h. kleiner als 1 mW, z.B. 0,5 mW. Sie dienen dazu, Informationen über den Zustand ihrer Umgebung zerstörungsfrei, berührungslos und mit hoher Auflösung zu gewinnen.

Die Sendeantennen S1 und S2 senden solche breitbandigen elektromagnetischen Pulse geringer Leistung und kurzer Dauer, z.B. weniger als eine Nanosekunde, aus. Treffen diese auf den Patienten P, so dringen sie in den menschlichen Körper ein und werden an aufeinanderfolgenden Grenzschichten verschiedener Gewebearten teilreflektiert. Solche Grenzschichten ergeben sich z.B. durch den Übergang von einem Organ zu einem anderen Organ. Mit den Empfangsantennen E1 und E2 werden die reflektierten Signale aus unterschiedlichen Tiefen des Körpers detektiert. Da die verschiedenen menschlichen Gewebearten typische Absorptions- und Reflexionseigenschaften haben, kann die Lage der Organe des Patienten P genau detektiert werden.

Wie bereits beschrieben wird vor Beginn der Bestrahlung die Position des Tumors T mittels Computertomographie CT überprüft. Zu diesem Zeitpunkt sollten auch Messungen mit den Radarsystemen erfolgen, so dass hiermit ein Sollmessergebnis der Radarmessungen bekannt ist.

Bewegungen des Patienten P, sowie dessen Atmung und Herzschlag verschieben und verformen die Grenzschichten und verändern damit das gemessene Signal der Radarsysteme. Durch geeignete Algorithmen kann hieraus in der Auswerteeinheit A die Verschiebung der Organe des Patienten P ermittelt werden. Es werden also aus den anatomischen Bewegungen Messdaten gewonnen, woraus die Organbewegungen ortsabhängig rekonstruiert werden kann. Durch den Einsatz der UWB-Radarsysteme ist es also möglich, die Organbewegungen in der Tiefe des Körpers kontaktlos festzustellen. Es ergibt sich eine zeitliche Abfolge von 3D-Bildern. Da die Position des Tumors T bezüglich der Organe bekannt ist, kann hieraus die aktuelle Position des Tumors T ermittelt werden.

Hierfür reicht in Prinzip bereits ein einzelnes UWB-System aus. Die Verwendung zweier oder mehrerer Systeme ist jedoch vorteilhaft, denn hierdurch werden redundante Daten zur Erhöhung der Sicherheit und Verbesserung der Ortsauflösung gewonnen. In Figur 1 ist der vorteilhafte Fall dargestellt, dass die beiden Sende-Empfänger-Systeme in einem Winkel von 45 Grad zueinander an der Decke des Behandlungsraums angebracht sind.

Die Auswerteeinheit A ermittelt aus den Daten der Empfänger E1 und E2 Signale, welche der Bewegung des Tumors T entsprechen. Diese können zur Steuerung des Ionenstrahls IS mittels der Steuereinrichtung S verwendet werden. Hierdurch kann der Ionenstrahl IS dem Tumor nachgeführt werden, so dass keine Fehlbestrahlung des Patienten P erfolgt. Es kann also der Ionenstrahl IS der Bewegung des kranken Organs folgen und den Tumor T zerstören, ohne gesundes Gewebe zu treffen. Alternativ hierzu ist es auch möglich, den Ionenstrahl abzuschalten, sobald eine Organ- oder Patientenbewegung mittels der Radarsysteme erkannt wurde.

Neben der beschriebenen Anwendung der Beobachtung der Lage des Tumors T können die Radarsysteme zur anderweitigen Überwachung eingesetzt werden:

Lebenswichtige Funktionen des Patienten P, wie Atmung und/oder Herzschlag, können mit Hilfe der Radarsysteme nichtinvasiv überwacht werden. Dies erfolgt durch eine Beobachtung des Brustkorbs mittels der Radare. Andere Patientenmonitore, wie z.B. EKG-Geräte, sind somit im Behandlungsraum während der Bestrahlung nicht vonnöten. Dies ist insofern vorteilhaft, als im Behandlungsraum befindliche Geräte während der Bestrahlung des Patienten P von Streustrahlung getroffen werden können, so dass Strahlenschäden drohen. Die Geräte sind dann radioaktiv, so dass sie erst "freigemessen" werden müssen, bevor sie den Behandlungsraum wieder verlassen dürfen. D.h., bevor ein solches Gerät aus dem Behandlungsraum entfernt wird, muss sichergestellt werden, dass es nicht (mehr) radioaktiv strahlt.

Eine durch die Radarsysteme festgestellte Änderung von Herzschlag und/oder Atmung kann dazu führen, dass der Ionenstrahl IS unterbrochen wird. Dies kann z.B. bei einem Panikzustand des Patienten P der Fall sein, welcher sich durch stark veränderte Atmung und Herztätigkeit bemerkbar macht.

Weiterhin ergibt sich die Möglichkeit, ein unbeabsichtigtes Annähern einer Person oder eines Gerätes an den Ionenstrahl IS oder an empfindliche elektronische Teile zu verhindern. Mit den Radarsystemen kann z.B. die Austrittsöffnung des Ionenstrahls überwacht werden. Nähert sich jemand oder etwas dieser Öffnung, sollte der Ionenstrahl IS bzw. eine Elektronikeinheit unterbrochen werden. Zusätzlich oder alternativ kann ein Alarm ausgelöst werden.

Schließlich kann auch die Umgebung von beweglichen Komponenten mit den Radarsystemen überwacht werden. In Betracht kommen hierbei z.B. Roboter oder ein Rangeshifter. Wird erkannt, dass sich eine Person oder eine Gerät in eine Zone bewegt hat, in welcher die Gefahr einer Kollision mit der überwachten beweglichen Komponente besteht, kann die Bewegung dieser Komponente gestoppt und/oder ein Alarm ausgelöst werden.

Grundsätzlich ist die Überwachung aller Geräte und/oder Personen möglich, welche sich innerhalb des Erfassungsbereiches der UWB-Radarsysteme befinden. Dieser beträgt typischerweise einige Meter im Durchmesser, d.h. bei Fokussierung auf den Patienten P einige Meter um den zu bestrahlenden Patienten P.

Die Erfindung wurde voranstehend an einem Ausführungsbeispiel beschrieben. Es versteht sich, dass zahlreiche Änderungen und Modifikationen möglich sind, ohne dass der Rahmen der Erfindung verlassen wird.

## Patentansprüche

1. Vorrichtung zur Bestrahlung eines Patienten (P), umfassend
eine auf den Patienten (P) ausgerichtete Bestrahlungsquelle, und
zumindest ein Radarsystem (S1, E1, S2, E2) zur Erfassung der Position des Patienten (P) oder eines Teils (T) des Patienten (P) während der Bestrahlung.

2. Vorrichtung nach Anspruch 1, wobei
es sich bei dem mindestens einen Radarsystem (S1, E1, S2, E2) um ein UWB-Radarsystem handelt.

3. Vorrichtung nach einem der Ansprüche 1 bis 2, mit
einer Auswerteeinheit (A) zur Ermittlung der Position des Patienten (P) oder des Teils (T) des Patienten (P) aus von dem mindestens einen Radarsystem (S1, E1, S2, E2) erfassten Daten.

4. Vorrichtung nach einem der Ansprüche 1 bis 3, wobei
es sich bei der Bestrahlungsquelle um eine Partikel emittierende Quelle handelt.

5. Vorrichtung nach einem der Ansprüche 1 bis 4, mit zwei Radarsystemen (S1, E1, S2, E2).

6. Vorrichtung nach einem der Ansprüche 1 bis 5, mit
einer Steuereinrichtung (S) zum Nachführen und/oder Unterbrechen des von der Bestrahlungsquelle emittierten Strahls aufgrund der erfassten Position des Patienten (P) oder des Teils (T) des Patienten (P).

7. Vorrichtung nach einem der Ansprüche 1 bis 6, mit
einer Auswerteeinheit (A) zur Ermittlung der Position einer Person und/oder eines Gerätes aus von dem mindestens einen Radarsystem (S1, E1, S2, E2) erfassten Daten.

8. Vorrichtung nach Anspruch 7, mit
einer Steuereinrichtung (S) zum Nachführen und/oder Unterbrechen des von der Bestrahlungsquelle emittierten Strahls aufgrund der ermittelten Position der Person und/oder des Gerätes.

9. Vorrichtung nach einem der Ansprüche 1 bis 8, mit
einer Auswerteeinheit (A) zur Ermittlung von einer Atem- und/oder Herztätigkeit des Patienten (P) aus von dem mindestens einen Radarsystem (S1, E1, S2, E2) erfassten Daten.

10. Vorrichtung nach Anspruch 9, mit
einer Steuereinrichtung (S) zum Nachführen und/oder Unterbrechen des von der Bestrahlungsquelle emittierten Strahls aufgrund der ermittelten Atem- und/oder Herztätigkeit.

11. Computerprogramm,
mit einem Eingang für Daten von zumindest einem Radarsystem (S1, E1, S2, E2) zur Erfassung der Position eines Patienten (P) oder eines Teils (T) des Patienten (P) während einer Bestrahlung,
mit einem Auswertebestandteil zur Ermittlung der Position des Patienten (P) oder des Teils (T) des Patienten (P) aus den Daten.

12. Computerprogramm nach Anspruch 11, mit
einem Steuerbestandteil zum Steuern des Nachführens und/oder Unterbrechens des von der Bestrahlungsquelle emittierten Strahls aufgrund der ermittelten Position des Patienten (P) oder des Teils (T) des Patienten (P).

13. Computerprogramm nach einem der Ansprüche 11 bis 12, mit einem weiteren Auswertebestandteil
zur Ermittlung der Position einer Person und/oder eines Gerätes und/oder
zur Ermittlung von einer Atem- und/oder Herztätigkeit des Patienten (P)
aus den Daten.

14. Verfahren zur Positionserfassung,
bei dem zumindest ein Radarsystem (S1, E1, S2, E2) zur Erfassung der Position eines Patienten (P) oder eines Teils (T) des Patienten (P) während einer Bestrahlung eingesetzt wird.
